(19)

Europäisches
Patentamt
European
Patent Office
Office européen
des brevets

(11) **EP 4 737 887 A2**

(12) **EUROPEAN PATENT APPLICATION**

(43) Date of publication:
**06.05.2026 Bulletin 2026/19**

(21) Application number: **25207497.6**

(22) Date of filing: **08.10.2025**

(51) International Patent Classification (IPC):
**G01N 21/64** (2006.01)     **G01N 21/82** (2006.01)
**G01N 21/47** (2006.01)

(52) Cooperative Patent Classification (CPC):
**G01N 21/6408; G01N 21/47; G01N 21/6445;
G01N 21/645; G01N 21/82; G01N 33/53;
G01N 35/00;** G01N 2021/4707; G01N 2021/4792;
G01N 2021/6421; G01N 2021/6441;
G01N 2021/825

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **11.10.2024 JP 2024179255**

(71) Applicant: **Canon Medical Systems Corporation
Otawara-shi, Tochigi 324-0036 (JP)**

(72) Inventors:
• **MASUMURA, Takahiro**
  **Tokyo (JP)**
• **NAKAMURA, Tomohiro**
  **Tokyo (JP)**
• **KAKEGAWA, Norishige**
  **Tokyo (JP)**

(74) Representative: **Marks & Clerk LLP
15 Fetter Lane
London EC4A 1BW (GB)**

(54) **AUTOMATED ANALYTICAL APPARATUS AND AUTOMATED ANALYTICAL METHOD**

(57)     An automated analytical apparatus of an embodiment includes a light source unit (10), a reaction vessel (30), a detection unit (40), and a processor (70). The light source unit (10) emits at least two lights having different wavelengths. The reaction vessel (30) contains a reaction solution containing a mixture of a measurement object and a reagent. The detection unit (40) receives at least two emitted light of first emitted light and second emitted light having different wavelengths emitted from the reaction vessel (30) by radiating incident light emitted from the light source unit (10) to the reaction vessel (30). The processor (70) calculates a concentration of the measurement object based on a signal output from the detection unit (40). The first emitted light is fluorescence obtained by wavelength conversion of the incident light by the reagent. The detection unit (40) has a separation means (55) for separating the first emitted light and the second emitted light and receiving the separated first emitted light and second emitted light through photodetectors (58,68). The processor (70) calculates the concentration of the measurement object from at least one of two output signals output from the detection unit (40) in response to the first emitted light and the second emitted light.

FIG. 5

EP 4 737 887 A2

## Description

FIELD

Embodiments disclosed in this specification and drawings relate to an automated analytical apparatus and an automated analytical method for analyzing the components of a test sample using antigen-antibody reactions.

BACKGROUND

In sample testing methods using antigen-antibody reactions, fluorescence polarization using the polarization properties of fluorescence has been known. Fluorescence polarization radiates linearly polarized excitation light to a mixture (reaction solution) of a test sample containing a test item (measurement object) and a fluorescent reagent, measures the fluorescence intensity emitted from the reaction solution by polarization resolution, and evaluates the degree of polarization (polarization anisotropy or anisotropy). The value of this anisotropy is highly sensitive to the rotational motion of the measurement object, and the rotational motion depends on the size of the measurement object. In antigen-antibody reactions, when a measurement object (antigen) is mixed with an antibody-modified reagent, the antigen and antibody react specifically and bind, forming aggregates.

Therefore, it is possible to detect changes in the size of a measurement object (agglutination reaction) with high sensitivity by measuring anisotropy. The relationship between change in the size of a measurement object and the measured anisotropy depends on the concentration relationship between the measurement object and a reagent. If the relationship between the concentration of the measurement object and the measured anisotropy is obtained in advance as a calibration curve using known amounts of reagent, it is possible to calculate the concentration of the measurement object from anisotropy measurement results. Patent Document 1 (Japanese Patent No. 1692254) discloses an analytical apparatus that uses this fluorescence polarization. Patent Document 2 (Japanese Patent No. 6013796) discloses an apparatus that analyzes agglutination reactions by measuring the intensity of transmitted light or scattered light emitted from a reaction solution. Furthermore, Patent Document 3 (Japanese Unexamined Patent Application, First Publication No. 2007-120976) discloses an apparatus that performs analysis by simultaneously measuring fluorescence polarization and the intensity of transmitted light or scattered light.

SUMMARY

According to fluorescence polarization disclosed in Patent Document 1, it is possible to detect a measurement object of a very low concentration with high sensitivity by optimally adjusting the amount of a reagent to be mixed in. However, under such conditions in which the reagent is adjusted for highly sensitive detection, the measurable concentration range is limited to a low concentration range. If the concentration of the measurement object exceeds this concentration range, the anisotropy value saturates at a constant value regardless of the concentration of the measurement object, resulting in no sensitivity to the concentration of the measurement object. On the other hand, adjusting the amount of reagent to enable measurement in a high-concentration range reduces measurement sensitivity in a low-concentration range. Thus, the fluorescence polarization has the problem of being unable to achieve both high sensitivity and a wide concentration range.

On the other hand, Patent Document 2 discloses an automated analytical method that simultaneously measures transmitted light and scattered light and selects the optimal measurement method depending on the concentration range of a measurement object. That is, by combining two measurement methods with different sensitivities, the measurement range can be extended. However, Patent Document 2 does not disclose fluorescence polarization. Furthermore, as pointed out in the text of Patent Document 2, if it is not known which measurement method can be used to measure with high accuracy in which concentration range, there is no value in combining different measurement methods.

Patent Document 3 also discloses an immunoassay that simultaneously measures fluorescence polarization and the intensity of transmitted or scattered light to calculate the modification rate of a modified protein. However, in Patent Document 3, since a portion of the scattered light that is not wavelength-converted is received by a fluorescence polarization detector, the modified protein is detected at an apparently higher level, making it impossible to calculate the modification rate with high accuracy.

An object to be achieved by embodiments disclosed in this specification and drawings is to provide an automated analytical apparatus and an automated analytical method capable of analyzing the concentration of a measurement object with high sensitivity over a wide range from low to high concentrations. However, the object to be achieved by the embodiments disclosed in this specification and drawings is not limited to the above object. Objects corresponding to the effects of each configuration shown in the embodiments which will be described below can also be positioned as other objects.

(1) An automated analytical apparatus of an embodiment includes a light source unit, a reaction vessel, a detection unit, and a processor. The light source unit emits at least two lights having different wavelengths. The reaction vessel contains a reaction solution containing a mixture of a measurement object and a reagent. The detection unit receives at least two emitted light of first emitted light and second emitted light having different wavelengths emitted from the reaction vessel by radiating incident light emitted from the light source unit to the reaction vessel. The processor calculates a concentration of the measurement object based on a signal output from the detection unit. The first emitted light is fluorescence obtained by wavelength conversion of the incident light by the reagent. The detection unit has a separation means for separating the first emitted light and the second emitted light and receiving the separated first emitted light and second emitted light through photodetectors. The processor calculates the concentration of the measurement object from at least one of two output signals output from the detection unit in response to the first emitted light and the second emitted light.

(2) In the automated analytical apparatus according to the aspect of (1), the processor may be configured to: evaluate signal changes in the output signals after a given time has elapsed since the start of a reaction in which the measurement object and the reagent are mixed; calculate a first signal change based on the first emitted light and a second signal change based on the second emitted light; and calculate the concentration of the measurement object based on the first signal change when the first signal change satisfies a predetermined condition, and calculate the concentration of the measurement object based on the second signal change when the first signal change does not satisfy the predetermined condition.

(3) In the automated analytical apparatus according to the aspect of (2), the reagent may include scattering particles containing fluorescent molecules, the first signal change may be a signal change based on fluorescence anisotropy, and the second signal change may be a signal change based on scattered light intensity or an autocorrelation function calculated from temporal fluctuations in scattered light intensity.

(4) In the automated analytical apparatus according to the aspect of (2), the reagent may contain at least two antibodies with different affinities, each labeled with a fluorescent molecule that emits light at a different wavelength, and the first signal change and the second signal change may be signal changes based on a fluorescence intensity of the fluorescence having a different wavelength.

(5) In the automated analytical apparatus according to any one of the aspects of (1) to (4), the separation means of the detection unit may be a dichroic mirror that reflects light having a longer wavelength of two lights having different wavelengths and transmits light having a shorter wavelength.

(6) In the automated analytical apparatus according to any one of the aspects of (1) to (3), the light source unit may have a linear polarizer, and at least one of the lights emitted from the light source unit may be linearly polarized and irradiates the reaction solution, and the detection unit may have a polarization separation element, separate the first emitted light emitted from the reaction solution by the linearly polarized light into a direction parallel to and a direction orthogonal to a polarization direction of the linearly polarized light, and receive the separated lights through the photodetectors.

(7) In the automated analytical apparatus according to the aspect of (5), desired light received by the photodetector may be incident on the dichroic mirror as s-polarized light and color-separated when A-C < C-B and incident on the dichroic mirror as p-polarized light and color-separated when A-C > C-B, C being a cut-off wavelength at which the reflectance of the dichroic mirror is 50%, A being the wavelength of light having the longer wavelength of the two lights having different wavelengths, and B being the wavelength of the light having the shorter wavelength.

(8) In the automated analytical apparatus according to any one of the aspects of (1) to (4), with respect to light intensities of the photodetector receiving the first emitted light and the photodetector receiving the second emitted light, an output of a first light source generating an emitted light with a lower light intensity may be greater than an output of a second light source, or a gain or exposure time of the photodetector receiving the emitted light with a lower light intensity may be greater than that of the other photodetector.

(9) The automated analytical apparatus according to any one of the aspects of (1) to (4) may further include a controller configured to alternately or sequentially turn on at least two light sources emitting lights having different wavelengths. The detection unit may alternately or sequentially receive the first emitted light and the second emitted light depending on turn-on times of the light sources.

(10) In the automated analytical apparatus according to any one of the aspects of (1) to (4), the lights emitted from the light source unit may be incident through a first surface of the reaction vessel and emitted through a second surface opposite the first surface.

(11) In the automated analytical apparatus according to the aspect of (2) or (3), the predetermined condition may include comparing the first signal change with the second signal change, and calculating the concentration of the measurement object based on concentration change of the measurement object or the larger signal change with respect to the elapse of time.

(12) An automated analytical method of an embodiment includes a radiation step of radiating at least two lights having different wavelengths to a reaction solution containing a mixture of a measurement object and a reagent specifically reacting with the measurement object; a light-receiving step of separately receiving at least two emitted light of a first emitted light and a second emitted light having different wavelengths emitted from the reaction solution; and a processing step of calculating a concentration of the measurement object based on a signal output in the light-receiving step. The processing step calculates the concentration of the measurement object from at least one of a change in the signal based on the first emitted light and a change in the signal based on the second emitted light.

According to the present invention, it is possible to provide an automated analytical apparatus capable of analyzing the concentration of a measurement object with high sensitivity over a wide range from low to high concentrations.

BRIEF DESCRIPTION OF THE DRAWINGS

FIG. 1 is a diagram showing an example of a measurement range of fluorescence polarization.
FIG. 2 is a schematic diagram of states of a measurement object, fluorophore, and aggregates thereof in a reaction solution under different concentration conditions.
FIG. 3 is a schematic diagram of changes in signals obtained by two measurements, fluorescence polarization and scattering, with respect to the concentration of the measurement object.
FIG. 4 is a schematic diagram of a configuration of the photometric unit of an automated analytical apparatus in example 1.
FIG. 5 is a detailed diagram of a fluorescence polarization measurement system in example 1.
FIG. 6 shows the spectrum of light emitted from a reaction vessel in FIG. 5.
FIG. 7 shows film characteristics of a dichroic mirror in FIG. 5.
FIG. 8 shows the spectrum of light after separation by the dichroic mirror in FIG. 5.
FIG. 9 shows other film characteristics of the dichroic mirror in FIG. 5.
FIG. 10 shows the spectrum of light after separation by a dichroic mirror having other film characteristics in FIG. 5.
FIG. 11 is a schematic diagram of a reagent, a measurement object, and aggregates thereof in example 1.
FIG. 12 is a diagram showing a measurement and analysis flow in example 1.
FIG. 13 is a schematic diagram showing an anisotropic signal obtained by fluorescence polarization and a depolarized signal obtained by scattering as a function of reaction time.
FIG. 14 is a detailed diagram of alternative 1 in example 1.

FIG. 15 shows the spectrum of light after separation by a dichroic mirror in FIG. 14.
FIG. 16 shows the spectrum of light after separation by a dichroic mirror having other film characteristics in FIG. 14.
FIG. 17 is a detailed diagram of alternative 2 in example 1.
FIG. 18 is a detailed diagram of alternative 3 in example 1.
FIG. 19 is a detailed diagram of alternative 4 in example 1.
FIG. 20 is a detailed diagram of alternative 5 in example 1.
FIG. 21 is a detailed diagram of a fluorescence measurement system in example 2.
FIG. 22 is a configuration diagram of an automated analytical apparatus in example 3.

DETAILED DESCRIPTION

An automated analytical apparatus and an automated analytical method according to an embodiment will be described below with reference to the drawings.

(Description of Principle and Problems of Automated Analytical Apparatus)

The automated analytical apparatus according to the present embodiment mixes a test sample, such as blood or urine collected from a subject such as a person, with a reagent and measures the concentration of a desired test item (measurement object) contained in the test sample. The reagent contains a fluorophore (fluorescent molecule), which is modified with an antibody. The antibody specifically reacts with an antigen (measurement object) contained in the test sample, causing the fluorophore-containing reagent to agglutinate via the antigen. By quantifying the degree of agglutination, the concentration of the measurement object can be measured. For this quantification, the automated analytical apparatus according to the present embodiment measures the polarization dependence of fluorescence intensity and calculates a parameter such as the degree of polarization (anisotropy) to evaluate the degree of agglutination. For example, a reaction solution is irradiated with linearly polarized excitation light, and a fluorescence intensity $I_{para}$ of polarized light parallel to the polarization direction of the excitation light, and a fluorescence intensity $I_{orth}$ of polarized light orthogonal to the polarization direction of the excitation light are measured with respect to the fluorescence emitted from the reaction solution. Using these two measurement results, the anisotropy r is calculated according to the following formula (1).

$$r = (I_{para} - I_{orth})/(I_{para} + 2I_{orth}) \ ... \ (\text{Formula } 1)$$

Alternatively, the anisotropy r may be calculated according to the following formula (2).

$$r = (I_{para} - I_{orth})/(I_{para} + I_{orth}) \ ... \ (Formula \ 2)$$

The fluorophore absorbs, excites, and emits light depending on the relative relationship between the polarization direction of the excitation light and the orientation (molecular axis) of the fluorescent molecules. The fluorophore undergoes rotational motion and translational motion due to Brownian motion in the reaction solution. During the fluorophore emission process, if the rotation of the fluorophore is sufficiently slower than the fluorescence lifetime and the effects of rotation are negligible, light is absorbed and emitted on the molecular axis parallel to the excitation light. Therefore, the fluorescence intensity measured is greatest for polarized light parallel to the excitation light. On the other hand, if the rotational motion of the fluorophore is much faster than the fluorescence lifetime, the fluorophore rotates randomly between light absorption and emission, and thus the measured fluorescence is unpolarized. In an intermediate state, if the fluorescence lifetime and rotational motion are roughly equivalent, the fluorophore emits light while maintaining the polarization direction of the excitation light to some extent even while the fluorophore rotates, resulting in polarization characteristics of the measured fluorescence. A key feature of fluorescence polarization is that, under these conditions, changes in the rotational motion of the fluorophore due to an agglutination reaction are ascertained by measuring the anisotropy of fluorescence. This rotational motion depends on the volume of the fluorophore (e.g., the cube of the size thereof), making it highly sensitive to changes in the size of the fluorophore. By utilizing this principle, it is possible to measure the degree of agglutination in a reaction solution with high sensitivity even for a measurement object at a very low concentration.

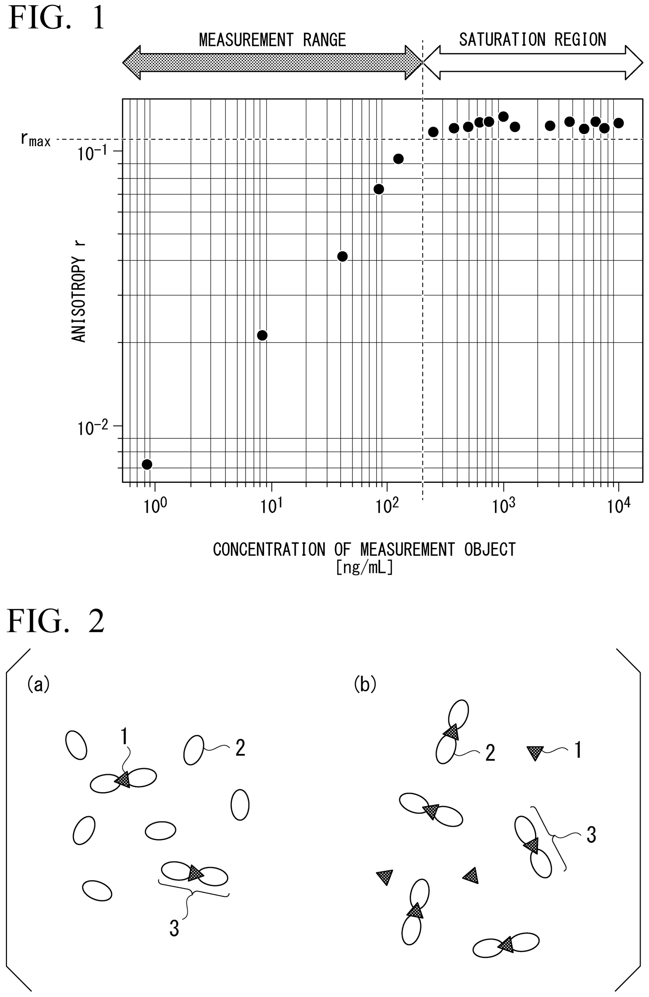

However, since there is an upper limit to the value of this anisotropy r, it is necessary to perform measurements within a range that does not reach the upper limit. FIG. 1 shows the results of measuring the concentration of a certain measurement object using fluorescence polarization. The horizontal axis represents the concentration of the measurement object in a reaction solution, and the vertical axis represents the anisotropy r. In FIG. 1, in the region where the anisotropy r changes depending on the concentration of the measurement object (the measurement range in the figure), there is a one-to-one correspondence between the measurement result of anisotropy r and the concentration of the measurement object, making it possible to calculate the concentration of the measurement object from the anisotropy r. However, when the anisotropy r exceeds a certain upper limit $r_{max}$, the anisotropy r becomes a constant value relative to the concentration of the measurement object (the saturation region in the figure), making it impossible to calculate the concentration from the value of the anisotropy. That is, if the value of the anisotropy r is below the upper limit $r_{max}$, the concentration can be quantified, and if it is above $r_{max}$, the anisotropy saturates and thus the concentration cannot be measured. In the example in FIG. 1, it can be ascertained that the concentration measurement range is roughly two orders of magnitude. The low concentration limit of the measurement range is determined by the S/N ratio of the measured fluorescence intensity.

It is possible to shift the measurement range toward higher concentrations by adjusting the amount of fluorophore reagent used in the reaction. However, in such a case, the measurement sensitivity on the lower concentration side sacrificed, making it impossible to take advantage of the features of fluorescence polarization.

The upper limit $r_{max}$ of the above-mentioned anisotropy r depends on the greater contribution of light emission from agglutinated fluorophores or light emission from unagglutinated fluorophores (hereafter referred to as "free fluorophores") to the measured fluorescence intensity. FIG. 2 is a diagram schematically showing states of a measurement object 1, fluorophores 2, and aggregates 3 thereof in a reaction solution. Rotation (rotational relaxation time) of free fluorophores 2 is assumed to be sufficiently shorter (faster) than the fluorescence lifetime of fluorophores 2. That is, when the fluorophore 2 is used alone, fluorescence becomes nearly unpolarized due to the effects of rotation, and the measured anisotropy is very small.

FIG. 2(a) shows a state in which the number of reagent fluorophores 2 is greater than the amount of the measurement object 1. In this case, the anisotropy is the sum of both contributions of light emission from free fluorophores 2 and light emission from agglutinated fluorophores (aggregates 3). This situation corresponds to a region in which the measured anisotropy changes depending on the concentration of the measurement object 1 under the condition of the measurement range shown in FIG. 1.

On the other hand, FIG. 2(b) shows a state in which the number of fluorophores 2 is less than the amount of the measurement object 1, and the measured fluorescence is almost entirely emitted from aggregates 3. The measured anisotropy is nearly a maximum possible value under those conditions. This is the state of the saturation region shown in FIG. 1. In this state, even if the concentration of the measurement object 1 becomes even higher, the measured fluorescence is still emitted from the aggregates 3, and thus the anisotropy value does not change.

On the other hand, scattered light scattered by the aggregates is affected by the anisotropy, and the polarization dependence of the scattered light changes. Therefore, the agglutination reaction can be evaluated by measuring the polarization dependency of scattered light (for example, the depolarized component of scattered light). As described in Patent Document 2, measurement of scattered light (hereinafter referred to as "scattering") can measure the measurement object at a low concentration with higher sensitivity than measurement of transmitted light (hereinafter referred to as "transmission"). On the other hand, in the present embodiment, a concentration range in which high-sensitivity measurement can be performed for both depolarization of scattered light by scattering and the anisotropy of fluorescence (fluorescence polarization) has been clarified through experiments.

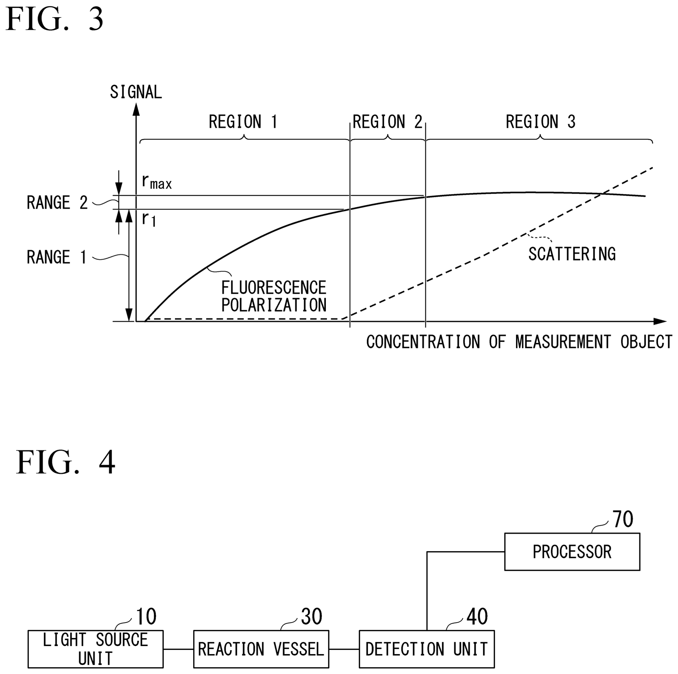

FIG. 3 is a schematic plot of experimental results, with the concentration of a measurement object plotted on the horizontal axis and a fluorescence polarization signal (change in anisotropy) and a scattering signal (change in the light intensity of a depolarized component) plotted on the vertical axis. As shown in FIG. 3, in the concentration range of region 1, fluorescence polarization is sensitive, and the signals fall within the measurement range shown in FIG. 1 (anisotropy $r < r_{max}$). In this low-concentration region, scattering lacks sufficient sensitivity and thus no significant signal is obtained. On the other hand, in the concentration range of region 2 (anisotropy $r_1 \leq r < r_{max}$), fluorescence polarization approaches the saturation region of FIG. 1 (anisotropy $r \geq r_{max}$) but is still within the measurement range. Furthermore, scattering enters a region where signal changes due to agglutination reaction can be gradually confirmed. Furthermore, in region 3, fluorescence polarization reaches the saturation region and is unable to measure changes in the concentration of the measurement object. On the other hand, scattering enters a region where signal changes can be confirmed sufficiently, allowing for accurate measurement of the concentration of the measurement object. From these results, it is possible to measure the concentration of a measurement object over a wide range even up to a relatively high concentration range by measuring a low-concentration measurement object using fluorescence polarization, switching to scattering when fluorescence polarization reaches the saturation region, and selecting the method with the better sensitivity in between, or using the results of both methods together.

Measurements based on dynamic light scattering (DLS) may be performed as scattering. In the reaction solution, the measurement object (scattering particles) and aggregates thereof move (translationally) in random directions due to Brownian motion. Small particles move relatively fast, while large particles move relatively slowly. Therefore, if temporal fluctuations in the intensity of scattered light emitted from the reaction solution are evaluated using an autocorrelation function, the former is observed as having a short correlation time, and the latter is observed as having a long correlation time. Generally, this autocorrelation function can be expressed as an exponential function such as $\exp(-\Gamma\tau)$ ($\tau$: delay time), and if the decay coefficient $\Gamma$ is used, the autocorrelation function for a short correlation time has a large

$\Gamma$, while the autocorrelation function for a long correlation time has a small $\Gamma$. Therefore, by quantifying the agglutination reaction based on this decay coefficient $\Gamma$, it is possible to measure the concentration of the measurement object. It is also confirmed that DLS can provide measurements with higher sensitivity than transmission through experiments, and a concentration range that can be measured is also confirmed. As a result, it is confirmed that it can be combined with fluorescence polarization similarly to depolarization of scattering. Therefore, scattered depolarized light, or DLS can be used as scattering. Alternatively, the intensity of scattered light emitted from the reaction solution at a specific angle may be measured.

Furthermore, the concentration range of measurement can be expanded by using two antibodies with different affinities for an antigen, labeling the antibodies with different fluorophores, and measuring the luminescence characteristics of the fluorophores (e.g., changes in luminescence intensity and anisotropy) due to binding in the antigen-antibody reaction over reaction time. For high-sensitivity measurements on the low concentration side, an emission signal from the high-affinity antibody is measured and analyzed, and for high concentrations, an emission signal from the low-affinity antibody is measured and analyzed. In this case, by measuring and analyzing fluorescent signals of at least two different emission wavelengths, it is possible to measure the concentration of the measurement object (antigen) over a wide range from low to high concentrations.

As described above, in the present embodiment, lights of two different wavelengths (fluorescence and scattered light, or two different fluorescences) are measured substantially simultaneously, and the two signals that change over reaction time are combined and analyzed to measure the concentration of the measurement object from low to high concentrations.

[Example 1]

FIG. 4 is a schematic diagram of a configuration of a photometric unit of an automated analytical apparatus in example 1. The detailed optical system will be described later. The photometric unit includes, for example, a light source unit 10, a reaction vessel 30, a detection unit 40, and a processor 70. The light source unit 10 includes a light source that emits light of two wavelengths. One wavelength excites fluorophores contained in a reagent, and the other wavelength is not absorbed by the fluorophores. The wavelength of the light source can be selected appropriately to match the excitation wavelength of the fluorophores in the reagent. For example, light in the visible range to the near-infrared range of wavelengths of 400 to 1100 nm may be used, or ultraviolet light with a wavelength of 400 nm or less, or infrared light with a wavelength of 1100 nm or more may be used. While an LED or laser can be used as the light source, it is preferable to use monochromatic light with a relatively narrow spectral width. The light source unit 10 is an example of a "light source unit." That is, the light source unit 10 emits at least two lights with different wavelengths.

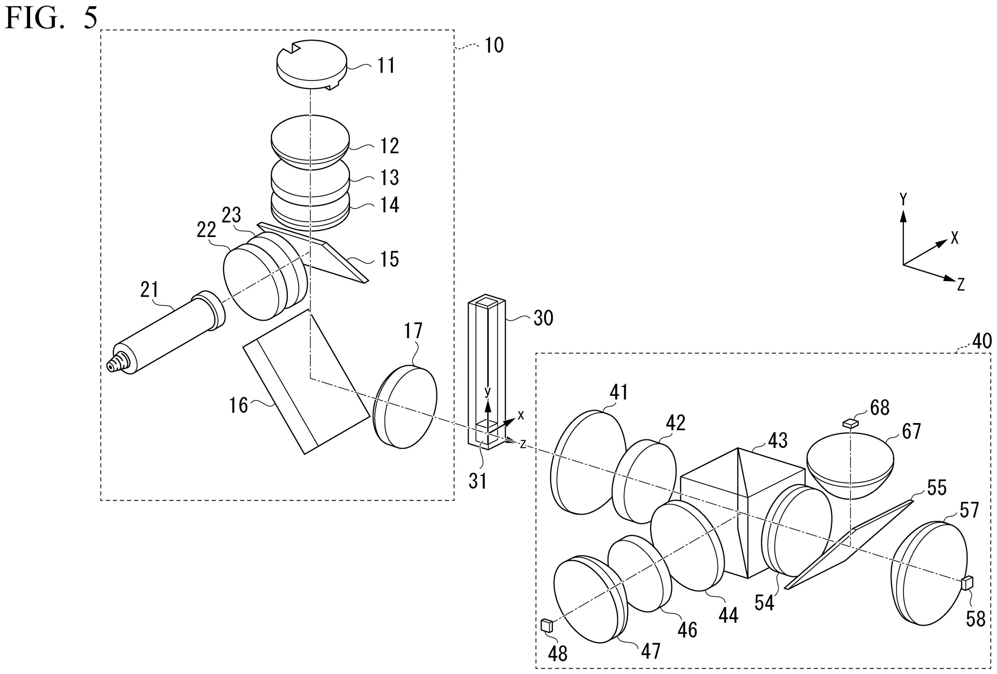

Lights with different wavelengths, which are emitted simultaneously from two light sources, pass through polarizers (linear polarizers) mounted on the light source unit 10 and are incident on the reaction solution 31 (FIG. 5) contained in the reaction vessel 30 as linearly polarized incident light. The reaction solution 31 is a mixture of a test sample containing a measurement object and a reagent containing fluorophores modified with an antibody that specifically reacts with the measurement object. In the reaction solution 31, aggregates are generated by an antigen-antibody reaction depending on conditions such as the sizes and concentrations of the measurement object and fluorophores, the time since mixing the measurement object and the reagent (reaction time), and the temperature of the reaction solution. After the test sample and the reagent are dispensed, the reaction solution 31 is stirred by a stirring unit (not shown), ensuring that the measurement object and the reagent are uniformly dispersed throughout the reaction solution. The reaction vessel 30 is an example of a "reaction vessel." That is, the reaction vessel 30 is capable of containing the reaction solution containing a mixture of the measurement object and the reagent that specifically reacts with the measurement object. Light emitted from the light source unit 10 enters the reaction vessel 30 from a first surface of the reaction vessel 30 and is emitted from a second surface opposite to the first surface.

The reaction vessel 30 emits fluorescence (first emitted light) emitted by the reagent when excited by the incident light, and non-excitation light (second emitted light) scattered by the reagent. These lights enter the detection unit 40, and the excitation light is cut off by an excitation light cut-off filter provided in the detection unit 40. Next, both the fluorescence and the scattered non-excitation light are separated by a polarizing beam splitter (hereinafter referred to as a "PBS") provided in the detection unit 40 into fluorescence and scattered light with polarization components parallel to polarization of the incident light (polarization-preserving components), and fluorescence and scattered light with polarization components orthogonal to the polarization of the incident light (depolarized components), and received by a detector provided in the detection unit 40. The received optical signals are sent to the processor 70, which can calculate anisotropy r from the fluorescence intensity and an autocorrelation function from the scattered light intensity, and output the concentration of the measurement object by referring to a previously prepared relationship between calculation results and concentrations of the measurement object (calibration curve). The detection unit 40 is an example of a "detection unit." That is, the detection unit 40 radiates the incident light emitted from the light source unit 10 to the reaction vessel 30 and receives the first emitted light and the second emitted lights with at least two different wavelengths emitted from the reaction vessel 30. The first emitted light is fluorescence obtained by wavelength-converting the incident light by the reagent. The detection unit 40 has a separation means for separating the first emitted light and the second emitted light and receiving the same through a photodetector.

The processor 70 realizes functions thereof by a hardware processor (computer) executing a program stored in a memory (not shown), for example. The hardware processor refers to circuitry such as a central processing unit (CPU), a graphics processing unit (GPU), an application specific integrated circuit (ASIC), a programmable logic device (e.g., a simple programmable logic device (SPLD) or a complex programmable logic device (CPLD)), or a field programmable gate array (FPGA).

The processor 70 is an example of a "processor." That is, the processor 70 calculates the concentration of the measurement object based on the signal output from the detection unit 40. The processor 70 calculates the concentration of the measurement object from at least one of the two output signals output from the detection unit 40 in response to the first emitted light and the second emitted light. The processor 70 evaluates signal changes in the output signals after a given time has elapsed since the start of reaction by mixing the measurement object and the reagent, calculates a first signal change based on the first emitted light and a second signal change based on the second emitted light, calculates the concentration of the measurement object based on the first signal change if the first signal change satisfies a predetermined condition, and calculates the concentration of the measurement object based on the second signal change if the first signal change does not satisfy the predetermined condition. The reagent includes scattering particles containing fluorescent molecules, the first signal change is based on fluorescence anisotropy, and the second signal change is based on scattered light intensity or an autocorrelation function calculated from temporal fluctuations in the scattered light intensity. In another signal change, the reagent contains at least two antibodies with different affinities, each labeled with a fluorescent molecule that emits light at a different wavelength, and the first signal change and the second signal change are based on the fluorescence intensities of fluorescence with different wavelengths. The predetermined condition is to compare the first signal change and second signal change and calculate the concentration of the measurement object based on the signal change that is larger with respect to the concentration change of the measurement object or the passage of time.

(Detailed Description of Fluorescence Polarization Measurement System)

A fluorescence polarization measurement system of example 1 will be described in detail using FIG. 5. Light emitted from the light source 11 is spread and is approximately collimated by a collimator lens 12. Light source 11 is an LED emitting excitation light with a wavelength of 340 nm.

The light that has passed through collimator lens 12 passes through a short-pass filter 13, a polarizer 14, and a dichroic mirror 15 that is positioned at an angle to transmit excitation light and reflect red light, and is reflected by a mirror 16 and focused by a condenser lens 17, irradiating the reaction solution 31 contained in the reaction vessel 30. At this time, the short-pass filter 13 has the characteristic of transmitting the excitation wavelength, and thus can block unnecessary light other than the desired wavelength received by the detection unit 40. In addition, the polarizer 14 transmits only light polarized in the z direction shown in FIG. 5, which is reflected by the mirror 16, and vertically polarized light (y direction) is then radiated to the reaction solution 31 as incident light. The z direction is the direction in which the condenser lens 17, the reaction vessel 30, and the collimator lens 41 are lined up in this order. In other words, the z direction is the direction in which light condensed by the condenser lens 17 travels toward the reaction vessel 30. The y direction is the vertical direction perpendicular to the z direction. The x direction is the direction perpendicular to the z-y plane.

The reaction solution 31 contained in the reaction vessel 30 contains the test sample described above, a reagent containing fluorophores, and aggregates thereof. When the reaction solution 31 is irradiated with the incident light, wavelength-converted fluorescence and excitation light that has not been wavelength-converted are emitted from the reaction solution 31. The central wavelength of the fluorescence is 611 nm.

The spread fluorescence and excitation light are approximately collimated by the collimator lens 41 and reach an excitation light cut-off filter 42. The excitation light cut-off filter 42 is designed to pass the desired wavelength received by the detection unit 40, and thus can cut off the 340 nm excitation light that has not been wavelength-converted. The fluorescence is then separated by a PBS 43 into horizontally (x-direction) polarized light and vertically (y-direction) polarized light. s-polarized light incident on the separation surface of the PBS 43 is reflected by the separation surface, passes through the polarizer 44 and a bandpass filter 46, which are positioned to maximize transmission of the reflected linearly polarized light, and is then focused by the condenser lens 47, and a detector 48 receives fluorescence intensity $I_{para}$ of the polarized component parallel to the polarization of the incident light. Therefore, the detector 48 receives only linearly polarized light in the y direction.

Furthermore, p-polarized light incident on the separation surface of the PBS 43 passes through the separation surface and then through the polarizer 54, which is positioned to maximize the transmission of linearly polarized light. Fluorescence with a wavelength of 611 nm passes through a dichroic mirror 55 positioned at an angle and is focused by a condenser lens 57, and a detector 58 receives fluorescence intensity $I_{orth}$ of the polarization component orthogonal to the polarization of the incident light. Therefore, the detector 58 receives only light linearly polarized in the x direction.

The anisotropy r can be calculated from the light intensities obtained from the two detectors 48 and 58 using formula (1) or (2) above. An avalanche photodiode (hereinafter referred to as "APD") is used for the detectors 48 and 58, but high-sensitivity optical sensors such as a photomultiplier tube (hereinafter referred to as a "PMT") and a multi-pixel photon counter (MPPC) may also be used.

(Detailed Description of Scattered Light Measurement System)

The light emitted from the light source 21, while smaller than that emitted from the light source 11, still is slightly spread and is approximately collimated by a collimator lens 22. The light source 21 is a semiconductor laser (hereinafter referred to as "LD") emitting red light with a wavelength of 635 nm.

The light that has passed through the collimator lens 22 passes through a polarizer 23, is reflected by the dichroic mirror 15, and, as in the fluorescence polarization measurement system described above, is reflected by the mirror 16 and focused by the condenser lens 17, irradiating the reaction solution 31 contained in the reaction vessel 30. At this time, the polarizer 23 transmits only light polarized in the z direction, and light polarized in the y direction is radiated to the reaction solution 31 as incident light.

The incident light is scattered by the reagent and aggregates in the reaction solution 31 and emitted from the reaction vessel 30 as scattered light.

The scattered light with spread is approximately collimated by a collimator lens 41 and passes through the excitation light cut-off filter 42. The scattered light that has passed through the excitation light cut-off filter 42 is then separated into horizontally polarized light and vertically polarized light by the PBS 43, similar to the fluorescence. The scattered light incident on the separation surface of the PBS 43 as s-polarized light is reflected by the separation surface, but this light is unnecessary for scattered light measurement and is cut off by the bandpass filter 46. This is because the detector 48 does not receive any light other than the desired fluorescence described above.

On the other hand, scattered light incident on the separation surface of the PBS 43 as p-polarized light passes through the separation surface and then through the polarizer 54. The scattered light that has passed through the polarizer 54 is then reflected by the dichroic mirror 55 and focused by a condenser lens 67, and a detector 68 receives scattered light intensity $I_{orth}$ of a polarization component orthogonal to polarization of the incident light. Therefore, the detector 68 receives only light linearly polarized in the x direction. An APD is used for the detector 68, but other highly sensitive optical sensors may also be used.

The dichroic mirror 55 is an example of a "separation means of a detection unit." That is, the dichroic mirror 55 reflects light with a longer wavelength between two lights with different wavelengths, and transmits light with a shorter wavelength. The PBS 43 is an example of a "polarization separation element." That is, the PBS 43 separates first emitted light emitted from the reaction solution by linear polarization into light parallel and light orthogonal to the polarization direction of the linearly polarized light, and receives each light through a photodetector.

(Effective Placement of Detector for Scattered Light Measurement)

The placement of the detector 68 in FIG. 5 will be described in detail below along with the spectrum in the optical system described above.

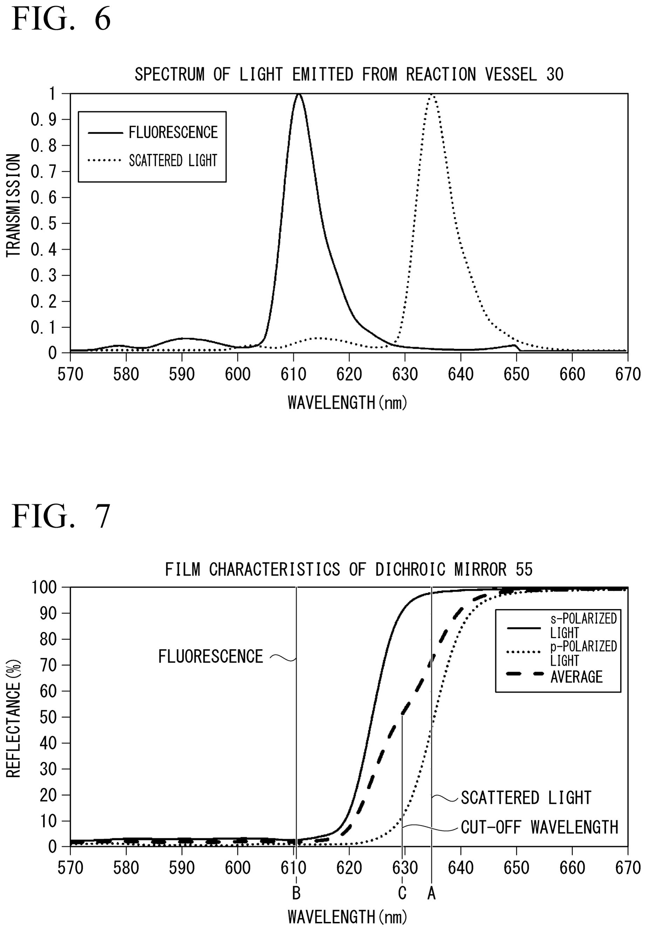

FIG. 6 shows the spectrum of light emitted from the reaction vessel 30. The solid line in FIG. 6 represents the spectrum of fluorescence with a center wavelength of 611 nm, which has been wavelength-converted by excitation light from the light source 11, and the dotted line represents scattered light with a center wavelength of 635 nm, emitted from the light source 21 and scattered by the reaction solution 31. Light with the spectrum of these two combined lights is emitted from the reaction solution 31.

FIG. 7 shows reflective film characteristics of the dichroic mirror 55. In FIG. 7, the solid line represents the reflectance of s-polarized light, the dotted line represents the reflectance of p-polarized light, and the dashed line represents the average reflectance. The wavelength at which the average reflectance is 50% is called the cut-off wavelength of this dichroic mirror.

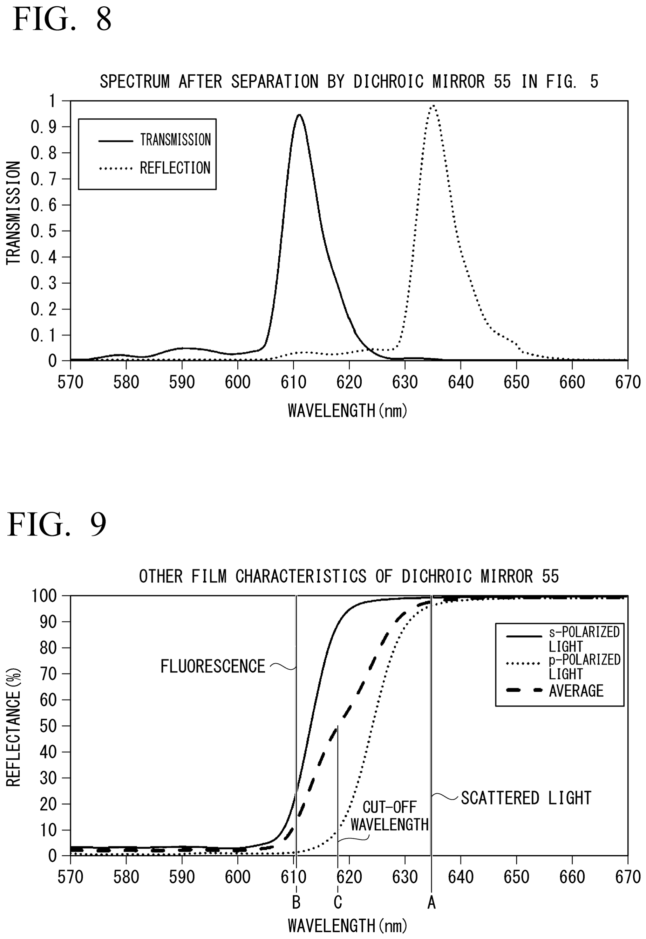

FIG. 8 shows the spectrum of light after separation by the dichroic mirror 55 in FIG. 5. The solid line represents light that has passed through the dichroic mirror 55, the dotted line represents light that has been reflected by the dichroic mirror 55, and these lights are focused by the condenser lenses 57 and 67 and received by the detectors 58 and 68. The desired light at the detector 58 is fluorescence with a central wavelength of 611 nm, and scattered light with a central wavelength of 635 nm, which could become noise light, is not received, and thus a highly accurate signal can be obtained. On the other hand, the desired light at the detector 68 is scattered light with a central wavelength of 635 nm, and fluorescence with a central wavelength of 611 nm, which could become noise light, is not received, and thus a highly accurate signal can be obtained here as well. If weak noise light is a problem, a bandpass filter (not shown in FIG. 5) may be provided in front of each condenser lens.

As such, light emitted from the reaction vessel 30 is composed of two lights with different wavelengths. If the central wavelength of the light with a longer wavelength (635 nm) is A, the central wavelength of the light with a shorter wavelength (611 nm) is B, and the cut-off wavelength mentioned above is C, optical arrangement in which light is incident as s-polarized light on a dichroic mirror (FIG. 7) having a characteristic of A-C < C-B, and a detector that obtains light with a desired wavelength (635 nm) is provided at a reflection position is the optimal arrangement (FIG. 5).

On the other hand, if a scattered light detector is provided at the transmission position of the dichroic mirror 55 and a fluorescence polarization detector is provided at the reflection position, as shown in FIG. 8, the scattered light detector mainly receives only fluorescence, whereas the fluorescence polarization detector mainly receives only scattered light, and thus it is necessary to provide a detector that receives long-wavelength light at the reflection position of the dichroic mirror 55.

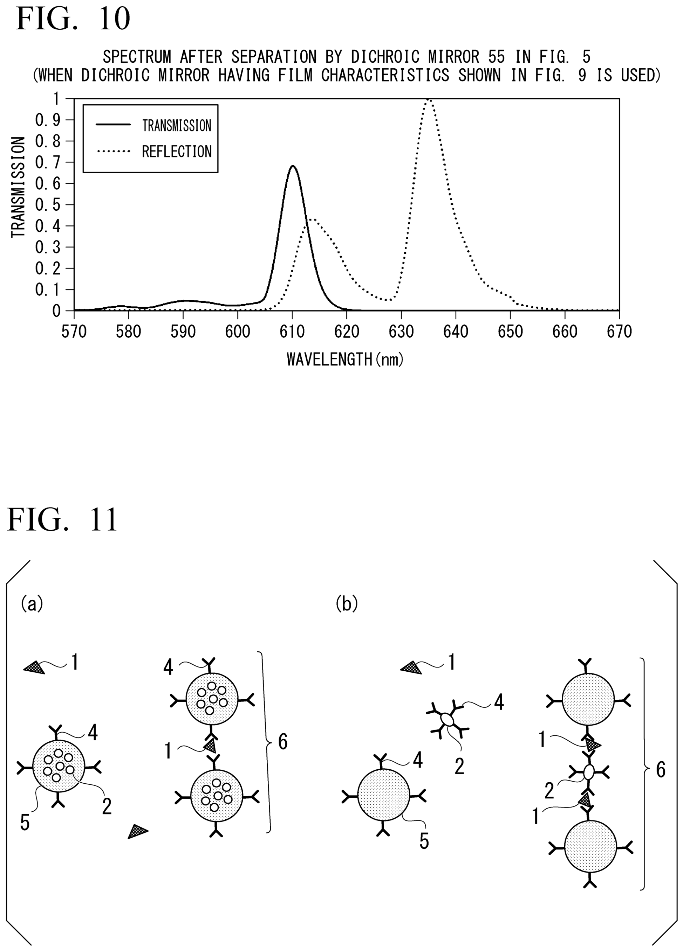

Next, a case in which a dichroic mirror having the film characteristics shown in FIG. 9 is used in the optical system of FIG. 5 is considered. The condition is A-C > C-B. In this case, the spectrum of light separated by the dichroic mirror is shown in FIG. 10. Since the light received by the detector 68 contains fluorescence, a bandpass filter (not shown) needs to be provided in front of the detector 68 to cut off the fluorescence.

(Description of Principle of Fluorescence Polarization Measurement)

The reagent used in this example will be described. Fluorescent molecules are typically small, making it difficult to accurately measure scattered light using a reagent composed solely of fluorescent molecules. In this regard, the reagent used in this example has a composition for measuring both fluorescence and scattered light, as shown below. FIG. 11 is a diagram schematically showing the reagent used in this example. FIG. 11(a) shows a reagent in which fluorescent molecules 2 are accumulated inside scattering particles 5. By modifying the scattering particles 5 with antibodies 4 that react specifically with the measurement object 1, both the fluorescent molecules and the scattering particles react specifically with the measurement object 1 to form aggregates 6.

When the aforementioned excitation light is radiated, the excitation light is absorbed by the fluorescent molecules 2, generating fluorescence. The rotational relaxation time of the fluorescent molecules changes before and after formation of the aggregates 6, resulting in change in the measured anisotropy of the fluorescence. On the other hand, when non-excitation light is radiated, the non-excitation light does not interact with the fluorescent molecules 2 and is scattered by the scattering particles 5. The polarization characteristics of the incident scattered light change depending on the scattering concentration of the reagent and the size and shape of the aggregates. That is, the intensity of the depolarized component of the scattered light changes. Thus, in this example, the reagent is provided with the functionality to measure two signals, fluorescence polarization and scattering, and lights with different wavelengths, excitation light and non-excitation light, are radiated, enabling two measurements. For example, by simultaneously illuminating lights of different wavelengths from the light source unit 10 and mixing the measurement object and the reagent, the two signals, the anisotropy of fluorescence and the depolarized component of the scattered light, can be measured at any time.

The reagent may also have the composition as shown in FIG. 11(b). Both the fluorescent molecules 2 and the scattering particles 5 are modified with antibodies 4 that react specifically with the measurement object 1. An antigen-antibody reaction generates aggregates 6 in which the measurement object 1 is sandwiched between the scattering particles 5 and the fluorescent molecules 2. The formation of aggregates slows the rotational relaxation time of the fluorophore, resulting in change in the anisotropy of the measured fluorescence. Similarly, the polarization characteristics of scattering also change due to the formation of aggregates. Therefore, as in the case of FIG. 11(a), this reagent can be used to simultaneously measure the anisotropy of the fluorescence and the depolarized component of the scattered light to evaluate the agglutination reaction.

The fluorophore 2 of the reagent can be appropriately selected and designed in consideration of characteristics such as absorption wavelength, emission wavelength, fluorescence emission efficiency, and fluorescence lifetime, and the combination with the antibodies 4 that specifically react with the measurement object 1. It is particularly desirable to use a fluorophore 2 having a fluorescence lifetime that is approximately the same order as the rotational relaxation time estimated from the size of the measurement object 1 and the size of the aggregates 6. The particles containing the fluorophore are luminescent particles that accumulate europium complexes, and latex particles having a diameter of 10 to 500 nm may be used, for example. The size and concentration of the scattering particles also affect the anisotropy of the fluorescence. Therefore, it is desirable to design the reagent in consideration of the S/N balance between the fluorescence polarization and the scattering.

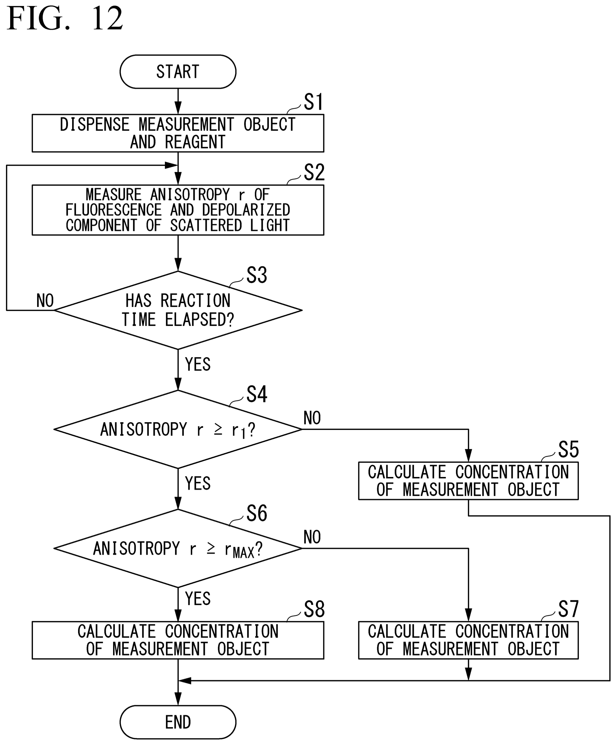

Next, FIG. 12 shows a measurement and analysis flow in this example. First, in step S1, a measurement object and a reagent are dispensed into the reaction vessel 30 to generate a reaction solution 31, and an agglutination reaction is initiated. Next, in step S2, excitation light and non-excitation light are radiated to the reaction solution 31, and the anisotropy r of fluorescence and the light intensity of the depolarized component of the scattered light are simultaneously measured. This measurement in S2 is performed continuously at a certain time interval with respect to the reaction time from the start of the agglutination reaction. In step S3, it is determined whether a predetermined reaction time has elapsed. The predetermined reaction time may be set to a time during which the agglutination reaction sufficiently converges. Alternatively, it may be set to any time (e.g., 5 minutes) within a range in which change in the reaction can be measured. Even if the reaction has not yet sufficiently converged, the concentration of the measurement object can be measured from the reaction speed by analyzing changes in signals within the time. If it is determined in step S3 that the predetermined reaction time has not elapsed, the measurement in S2 is repeated. The number of measurements performed within the reaction time can be set arbitrarily depending on an analysis process after the measurement.

If it is determined in step S3 that the predetermined reaction time has elapsed, the measurement ends and the process proceeds to the analysis flow of step S4 and subsequent steps. First, in step S4, the anisotropy r of the reaction solution 31 is evaluated on the basis of measurement results of fluorescence polarization. For example, the anisotropy r may be evaluated from the result of the last measurement within the reaction time. This anisotropy r is compared with a first predetermined value $r_1$. The predetermined value $r_1$ corresponds to $r_1$ shown in FIG. 3, for example. If the measured anisotropy r is less than $r_1$ (region 1 in FIG. 3, NO in step S4), the concentration of the measurement object is calculated from the calibration curve of fluorescence polarization on the basis of the result of the anisotropy r in step S5. For example, the solid line in FIG. 3 is the calibration curve (first calibration curve) of fluorescence polarization. Using standard samples including measurement objects with known concentrations, the correspondence between the concentration of the measurement object and the anisotropy value can be measured under the same measurement conditions as in step S1, and this can be used as the first calibration curve.

On the other hand, if the anisotropy r is equal to or greater than $r_1$ (YES in step S4) the process proceeds to step S6 in which the anisotropy r is compared with a second predetermined value $r_{max}$. This predetermined value $r_{max}$ corresponds, for example, to $r_{max}$ shown in FIG. 3. If the measured anisotropy r is less than $r_{max}$ (region 2 in FIG. 3, NO in step S6), the concentration of the measurement object is calculated in step S7. In step S7, the measurement results of both the fluorescence polarization and the scattering may be used to calculate the concentration from one of the respective calibration curves. Alternatively, a more sensitive measurement method may be selected on the basis of the results of a calibration curve previously measured depending on the measured signals, and the concentration of the measurement object may be calculated from the measurement results of the selected measurement method. For example, the dashed line in FIG. 3 is the calibration curve of the scattering (second calibration curve). The second calibration curve may also be obtained in advance using standard samples. In region 2 of FIG. 3, the first calibration curve and the second calibration curve are compared, and the curve with a sufficiently large signal-to-noise ratio and a large amount of change in signals relative to change in the concentration of the measurement object can be selected.

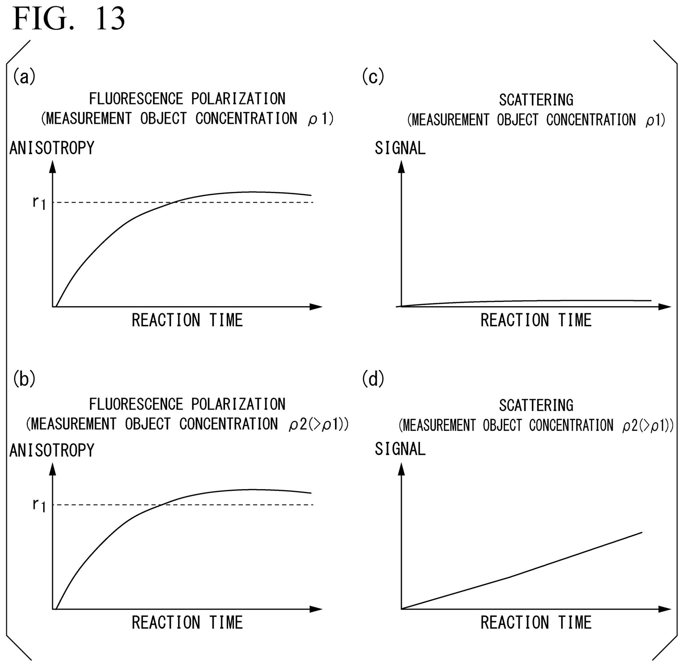

FIG. 13 is a diagram schematically showing the anisotropy signal measured by fluorescence polarization and a light intensity signal of the depolarized component measured by scattering as a function of reaction time. The condition for proceeding to step S7 is that the anisotropy r measured by fluorescence polarization satisfies $r_1 \leq r < r_{max}$. Since the measured anisotropy value is close to the saturation region shown in FIG. 1, even if the concentration of the measurement object is different (concentration $\rho 2$ > concentration $\rho 1$), fluorescence polarization produces almost identical results over reaction time, as shown in FIG. 13(a) and FIG. 13(b). On the other hand, the results of scattering corresponding to the fluorescence polarization results shown in FIG. 13(a) and FIG. 13(b) are as shown in FIG. 13(c) and FIG. 13(d). When the concentration of the measurement object is low, scattering does not produce sufficient signal change, resulting in the results shown in FIG. 13(c). In this case, the concentration may be calculated from the measurement results of fluorescence polarization shown in FIG. 13(a). On the other hand, when the concentration of the measurement object is greater than that shown in FIG. 13(c), sufficient signal change is obtained by scattering, as shown in FIG. 13(d). In this case, the concentration of the measurement object may be calculated on the basis of the measurement results of scattering. That is, the measurement method that produces large signal changes, i.e., is more sensitive to the concentration of the measurement object, can be selected from the two measurement results of fluorescence polarization and scattering.

Further, the predetermined values $r_1$ and $r_{max}$ may be determined on the basis of the first calibration curve (FIG. 1 or FIG. 3). Furthermore, $r_{max}$ may be determined on the basis of the saturation value of anisotropy. Alternatively, $r_{max}$ may be set to the anisotropy value corresponding to the concentration at which scattering is more sensitive than fluorescence polarization. In this case, $r_{max} = r_1$, and steps S6 and S7 may be omitted. If $r \geq r_1 (= r_{max})$ in step S4, the process proceeds to step S8. Alternatively, if the measurement result in step S6 is $r \geq r_{max}$ (YES in step S6), the concentration of the measurement object is calculated in step S8 using the measurement results of scattering.

Fundamentally, fluorescence polarization is more sensitive in the low-concentration range than scattering. Therefore, to maximize the use of high sensitivity of fluorescence polarization in the low-concentration range, the amount of reagent dispensed may be adjusted to match the measurement limit for the lowest concentration of the measurement object in the conditions for the aforementioned apparatus configuration. Further, the amount of reagent dispensed is adjusted such that fluorescence polarization covers the low-concentration range and scattering covers the high-concentration range, and is desirable for the measurement ranges to overlap. Furthermore, as a switching point between the two measurement methods, if the anisotropy is within the measurement range (region 1 in FIG. 3) (step S5), fluorescence polarization is used preferentially to calculate the concentration of the measurement object. Conversely, if the anisotropy is within the saturation region (region 3 in FIG. 3) (step S8), scattering is used. If the anisotropy is within region 2 in FIG. 3 (step S7), the method with higher sensitivity between fluorescence polarization and scattering is used in region 2. In this example, measurement results of both fluorescence polarization and scattering can be acquired, and appropriately selected during analysis in step S4 and subsequent steps.

Furthermore, in the automated analytical apparatus of this example, a separate light-receiving system can also be added to measure light having a polarization component (polarization-preserving component) parallel to polarization of incident light (non-excitation light). This is transmitted light that travels straight through without interacting with the reaction solution 31, and it is possible to measure the agglutination reaction as change in the intensity of the transmitted light. For example, depending on the concentration of the measurement object, three measurement results of fluorescence polarization (fluorescence), scattering (scattered light), and transmission (transmitted light) may be used. As noted in Patent Document 2, the transmission has lower sensitivity at low concentrations than the scattering, but can measure up to high concentrations. Therefore, it is possible to obtain measurement results using the three measurement methods up to step S3, and then analyze low concentrations using fluorescence polarization, medium concentrations using scattering, and high concentrations using transmission, according to a switching point based on a previously measured calibration curve. By combining the three measurement methods, it is possible to further expand the measurement range and improve accuracy. As an example, the concentration of the measurement object can be measured over a dynamic range of six orders of magnitude or more.

The calibration curve signal may not be the fluorescence anisotropy r or the light intensity of the depolarized component of the scattered light itself, but may be the amount of change from the start of the reaction. For example, fluorescence is evaluated by taking the anisotropy immediately after the start of the reaction as $r_0$, and evaluating change in the anisotropy from the start of the reaction as $\Delta r$ (= r - $r_0$), or $r/r_0$. On the other hand, scattered light may be evaluated as $\Delta I$ (= I - $I_0$), or $I/I_0$. $I_0$ and I are the light intensities of the depolarized component of the scattered light immediately after the start and thereafter.

Hereinafter, modified examples of the detection unit 40 in example 1 will be described.

(Alternative 1 of Example 1)

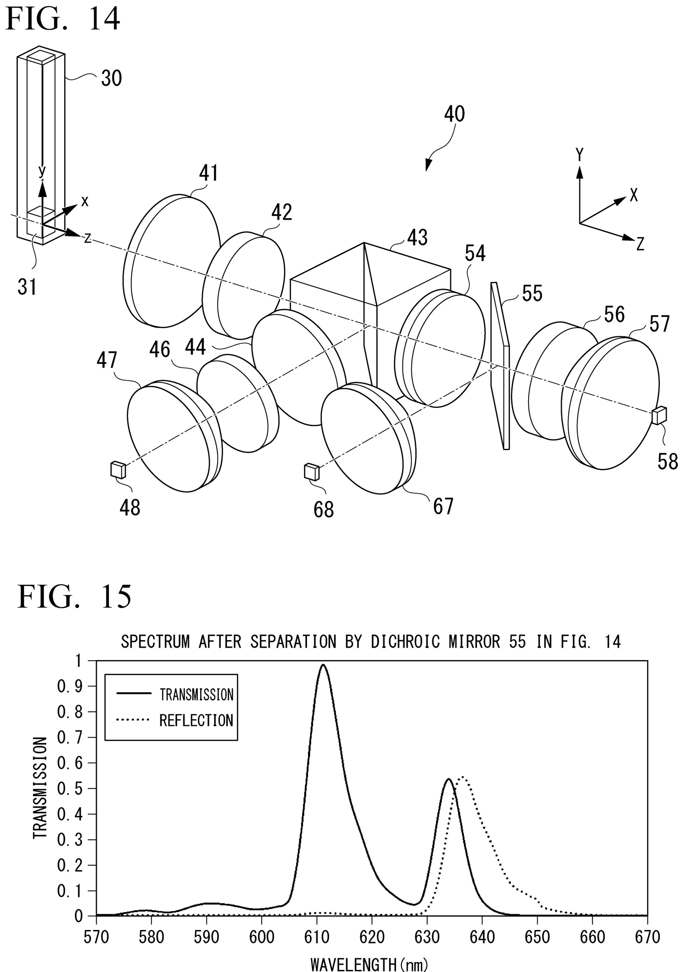

FIG. 14 shows the configuration of the detection unit 40 located after the reaction vessel 30 in alternative 1 of example 1. The structure is the same as that in FIG. 5 up to the polarizer 54. Linearly polarized light in the horizontal direction (x direction) that has passed through the polarizer 54 is incident on the dichroic mirror 55 as p-polarized light and is color-separated. The characteristics of the dichroic mirror 55 at this time are as shown in FIG. 7. Transmitted fluorescence passes through the bandpass filter 56, is focused by the condenser lens 57, and is received by the detector 58. On the other hand, the scattered light reflected by the dichroic mirror 55 is focused by the condenser lens 67 and is received by the detector 68.

FIG. 15 shows the spectrum of the light after separation by the dichroic mirror 55 in FIG. 14. Desired light at the detector 58 is fluorescence with a central wavelength of 611 nm, and scattered light with a central wavelength of 635 nm, which could become noise, is cut off by the bandpass filter 56. Meanwhile, desired light at the detector 68 is scattered light with a central wavelength of 635 nm, and fluorescence with a central wavelength of 611 nm, which could become noise, is sufficiently reduced and not received, and thus a highly accurate signal can be obtained without providing a bandpass filter.

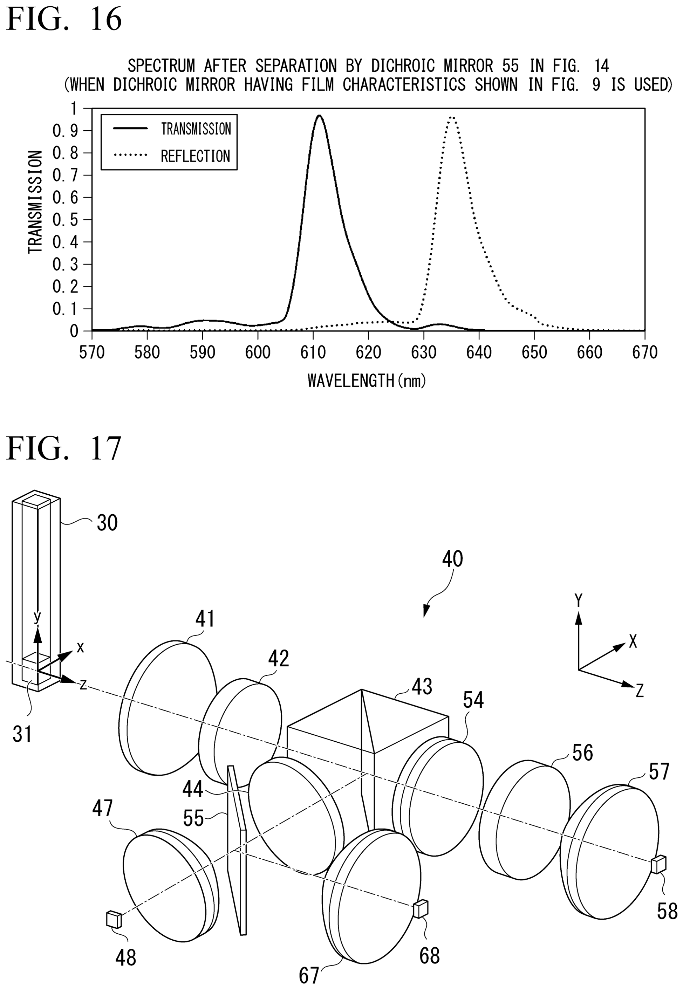

Further, FIG. 16 shows the spectrum of light after separation by the dichroic mirror 55 when the characteristics of the dichroic mirror 55 are changed to those shown in FIG. 9. In this case, fluorescence and scattered light are separated and incident on the detectors, and light that could become noise is sufficiently small, and thus the bandpass filter 56 shown in FIG. 14 may not need to be provided.

(Alternative 2 of Example 1)

FIG. 17 shows the configuration of the detection unit 40 located after the reaction vessel 30 in alternative 2 of example 1. The polarizer 14 in FIG. 5 is rotated 90° relative to the y-axis and transmits only light polarized in the x direction, which is reflected by the mirror 16, and the reaction solution 31 is irradiated with the light polarized in the x direction.

Light incident on the detection unit 40 is separated into horizontally polarized light and vertically polarized light by the PBS 43. s-polarized light incident on the separation surface of the PBS 43 is reflected from the separation surface and passes through the polarizer 44, which is located to maximize the transmission of the transmitted linearly polarized light. Fluorescence with a wavelength of 611 nm is incident on and passes through the dichroic mirror 55, which is positioned at an angle, as s-polarized light, is focused by the condenser lens 47, and is received by the detector 48. The characteristics of the dichroic mirror 55 at this time are as shown in FIG. 7. On the other hand, the scattered light reflected by the dichroic mirror 55 is focused by the condenser lens 67 and received by the detector 68.

The spectrum after separation by the dichroic mirror 55 is generally similar to that shown in FIG. 8, and noise in each detector is negligible. If the characteristics of the dichroic mirror 55 are as shown in FIG. 9, the spectrum is generally similar to that shown in FIG. 10, and a bandpass filter or long-pass filter that cuts off fluorescence with a wavelength of 611 nm need to be provided at the reflection position of the dichroic mirror 55.

(Alternative 3 of Example 1)

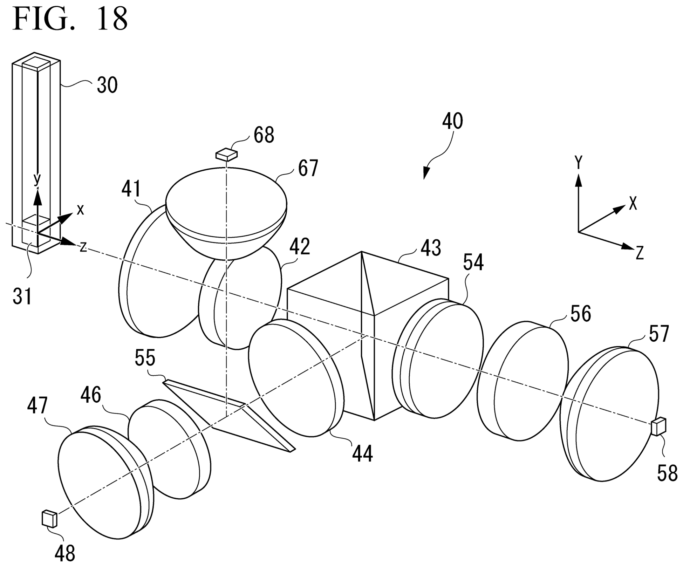

FIG. 18 shows the configuration of the detection unit 40 located after the reaction vessel 30 in alternative 3 of example 1. The light path for reflection of light from the PBS 43 is the same as that shown in FIG. 17 up to the polarizer 44.

Fluorescence with a wavelength of 611 nm is incident on and passes through the dichroic mirror 55 disposed at an angle as p-polarized light, passes through the bandpass filter 46, is focused by the condenser lens 47, and is received by the detector 48. At this time, the characteristics of the dichroic mirror 55 are as shown in FIG. 7. On the other hand, scattered light reflected by the dichroic mirror 55 is focused by the condenser lens 67 and is received by the detector 68.

Since the spectrum after separation by the dichroic mirror 55 is generally similar to that shown in FIG. 15, it is necessary to cut off the scattered light of 635 nm using the bandpass filter 46. When the dichroic mirror 55 has characteristics such that the relationship between A-C > C-B at the wavelengths described above is as shown in FIG. 9, the p-polarized light incident on the dichroic mirror 55 is separated, and the spectrum after separation is generally similar to that shown in FIG. 8, and noise at each detector is negligible.

(Alternative 4 of Example 1)

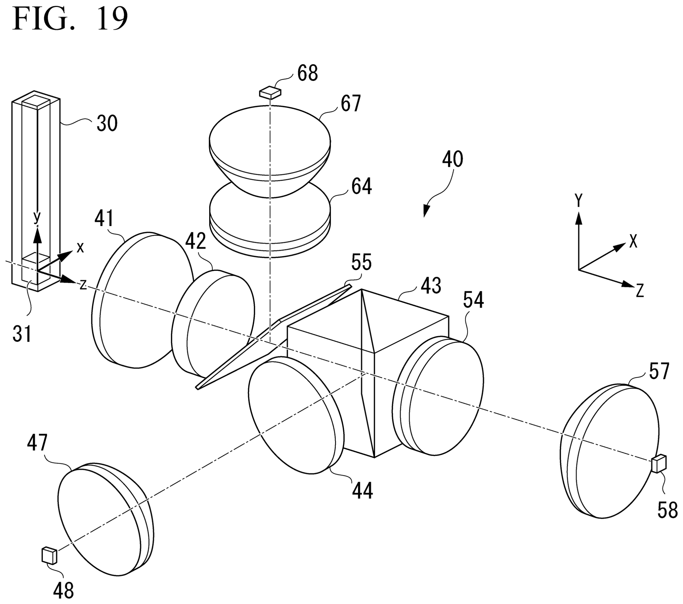

FIG. 19 shows the configuration of the detection unit 40 located after the reaction vessel 30 in alternative 4 of example 1. The configuration up to the excitation light cut-off filter 42 is the same as in FIG. 5.

Scattered light with a wavelength of 635 nm is reflected by the dichroic mirror 55 positioned at an angle, passes through the polarizer 64, is focused by the condenser lens 67, and is received by the detector 68. Desired polarized light at the detector 68 is light polarized in the x direction, and thus the polarizer 64 is positioned such that light polarized in the z direction does not pass through. In this case, if a dichroic mirror having the characteristics shown in FIG. 7 is used, the spectrum after separation by the dichroic mirror 55 is generally similar to that shown in FIG. 8. Next, fluorescence that has passed through the dichroic mirror 55 is separated into horizontally polarized light and vertically polarized light by the PBS 43, and the horizontally polarized light and vertically polarized light are received by detectors for fluorescence polarization measurement.

As mentioned above, a detector that receives light on the longer wavelength side needs to be disposed at the reflection position of the dichroic mirror 55. Furthermore, although two lights of different wavelengths are emitted from the reaction vessel 30, if the central wavelength on the longer wavelength side is A, the central wavelength on the shorter wavelength side is B, and the cut wavelength mentioned above is C, it is desirable to use a dichroic mirror having the characteristic of A-C < C-B when s-polarized light is incident on the dichroic mirror, and to use a dichroic mirror having the characteristic of A-C > C-B when p-polarized light is incident thereon.

(Alternative 5 of Example 1)

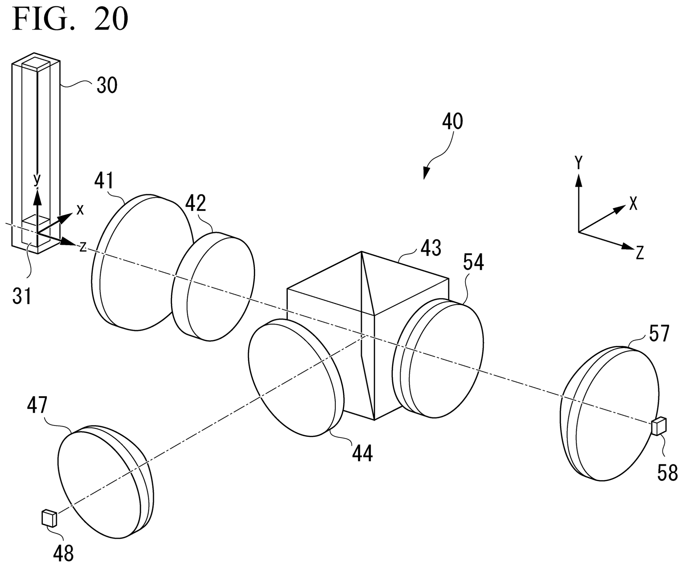

FIG. 20 shows the configuration of the detection unit 40 located after the reaction vessel 30 in alternative 5 of example 1. The light path through the PBS 43 is the same as that shown in FIG. 5 up to the polarizer 54.

Horizontally (x-direction) linearly polarized light that has passed through the polarizer 54 is focused by the condenser lens 57 and received by the detector 58. On the other hand, light reflected by the PBS 43 is vertically (y-direction) linearly polarized light that has passed through the polarizer 44, and is focused by the condenser lens 47 and received by the detector 48.

Since fluorescence and scattered light are separated by the PBS 43 based solely on the difference in polarization and received by the detectors, the light intensities $I_{para}$ and $I_{orth}$ obtained by simultaneously turning on light sources of different wavelengths are the sums of the light intensities $I_{Fpara}$ and $I_{Forth}$ of the fluorescence and the light intensities $I_{Spara}$ and $I_{Sorth}$ of the scattered light, respectively, and cannot be measured separately. Therefore, the light source 11 and light source 21 are controlled to be turned on alternately or sequentially, and the fluorescence and the scattered light are measured separately by the respective detectors accordingly.

For example, when the light source 11 is first turned on, fluorescence is emitted from the reaction vessel 30, and anisotropy can be obtained from the light intensities $I_{Fpara}$ and $I_{Forth}$ acquired by the detectors 48 and 58. Next, when the light source 21 is turned on, scattered light is emitted from the reaction vessel 30, and the scattered component becomes the light intensity $I_{Sorth}$ acquired by the detector 58. The detector 58 receives the fluorescence and scattered light alternately or sequentially, and thus can acquire two signals of fluorescence polarization and scattering almost simultaneously. The PBS used here is designed to handle fluorescence and scattered light of different wavelengths and can efficiently separate s-polarized light and p-polarized light.

Depending on the conditions of the reagent contained in the reaction solution 31 and the concentration of the measurement object, the intensity difference between the fluorescence and scattered light received by the detectors may increase, causing saturation of the light intensity of one of the detectors. Therefore, the output of the light source causing saturation is reduced compared to the other light source. Alternatively, saturation can be prevented by shortening the turn-on time of the light source causing saturation. Generally, as the concentration increases, the output of scattered light tends to increase, and thus the output of light source 21 is often reduced.

In the above, the light sources 11 and 21 are turned on sequentially, and the fluorescence and the scattered light are received alternately or sequentially by the detectors. At this time, signals generated from the light intensities of the fluorescence and the scattered light are output alternately or sequentially from the detectors.

As another method, an array sensor such as a CCD or a CMOS can be used as the detector. The array sensor is equipped with an RGB color filter, a wavelength cut filter, or a neutral density filter for each light-receiving region, and can simultaneously receive lights of different wavelengths emitted from the reaction solution. In this case, a neutral density filter can be disposed for each wavelength, and thus the above-mentioned control is unnecessary.

In this example, an LED or an LD is used as the light source, but it is also possible to use a superluminescent diode (SLD), which has a higher output than an LED and has an excitation wavelength suitable for the emission wavelength of fluorophore, a solid-state laser that emits YAG harmonics with a spectral width suitable for DLS, or a gas laser such as He-Ne.

Desired light received by the photodetector may be s-polarized light incident on the dichroic mirror and color-separated if A-C < C-B, where C is the cut-off wavelength at which the reflectance of the dichroic mirror is 50%, A is the wavelength of long-wavelength light between two lights with different wavelength, and B is the wavelength of short-wavelength light. If A-C > C-B, the light may be p-polarized light incident on the dichroic mirror and color-separated. Of the light intensities of the photodetector receiving the first emitted light and the photodetector receiving the second emitted light, the output of the first light source forming the emitted light with a lower light intensity may be greater than the output of the second light source, or the gain or exposure time of the photodetector receiving the emitted light with a lower light intensity may be greater than the other photodetector.

[Example 2]

(Fluorescence x Fluorescence)

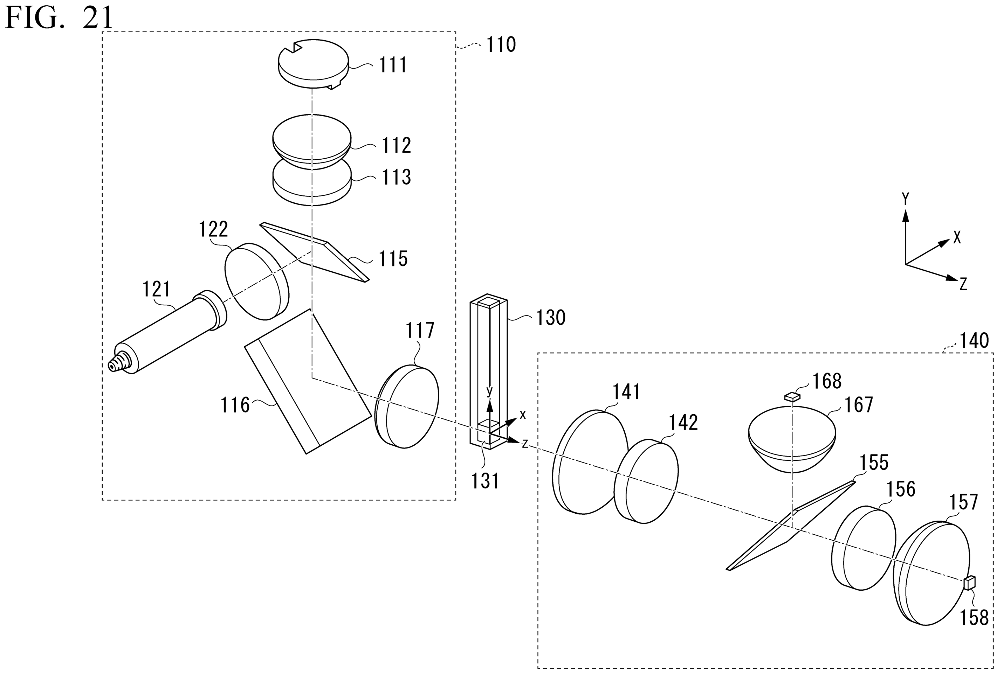

FIG. 21 shows the configuration of an automated analytical apparatus in example 2. The optical system is generally the same as in example 1, except that two lights with different wavelengths emitted from a light source unit 110 is radiated to a reaction solution 131.

The reaction solution 131 is composed of two antibodies with different affinities for antigens and different fluorescent labels corresponding thereto, and each fluorophore emits fluorescence of a different wavelength in response to the two excitation lights. In this example, changes in the emission intensities of the two fluorophores are measured by utilizing the difference in binding between antigen-antibody reactions over reaction time. For example, a method of reducing the distance between reagents by an agglutination reaction to cause energy transfer, and measuring the concentration of a measurement object by measuring change in the intensity of fluorescence generated due to the energy transfer (light enhancement or quenching) may also be applied. It is assumed that the reagents are mixed under appropriate conditions depending on the measurement method.

A measurement system that measures the concentration of a measurement object from changes in the emission intensities of two different fluorophores will be described in detail below.

(Detailed Description of Measurement of First Fluorescence (Short Wavelength side))

Light emitted from a light source 111 is approximately collimated by a collimator lens 112. As an example, the light source 111 is an LED having a wider spectral bandwidth than a laser. The light that has passed through the collimator lens 112 and a short-pass filter 113 passes through a dichroic mirror 115, is reflected by a mirror 116 and focused by a condenser lens 117 to irradiate a reaction solution 131 contained in a reaction vessel 30. The short-pass filter 113 allows an excitation wavelength to pass through, thereby cutting off any unnecessary light other than light with a desired wavelength emitted by the LED and received by a detection unit 140.

First fluorescence corresponding to the excitation wavelength is emitted from the reaction vessel 30. This is fluorescence with the shorter wavelength of the two fluorescences with different wavelengths. The emitted fluorescence having a spread is approximately collimated by the collimator lens 141 and passes through an excitation light cut-off filter 142. The fluorescence then passes through a dichroic mirror 155 positioned at an angle and a bandpass filter 156, is focused by a condenser lens 157, and is received by a detector 158.

(Detailed Description of Measurement of Second Fluorescence (Long Wavelength Side))

Light emitted from a light source 121 is approximately collimated by a collimator lens 122. As an example, the light source 121 is an LD with a narrow spectral bandwidth. The light that has passed through the collimator lens 122 is reflected by the dichroic mirror 115, and then is reflected by the mirror 116 and focused by the condenser lens 117, as described above, to irradiate the reaction solution 131 contained in the reaction vessel 30.

Second fluorescence corresponding to the excitation wavelength is emitted from the reaction vessel 30. This is fluorescence with the longer wavelength of the two fluorescences with different wavelengths. The emitted second fluorescence having a spread, like the first fluorescence, is approximately collimated by the collimator lens 141 and passes through the excitation light cut-off filter 142. The second fluorescence is then reflected by the dichroic mirror 155, focused by a condenser lens 167, and received by a detector 168.

(Arrangement Effective for Fluorescence Measurement)

The arrangement after the dichroic mirror 155 in FIG. 21 will be described in detail. The light emitted from the reaction vessel 130 becomes two fluorescences with different wavelengths. When the center wavelength of the second fluorescence is A, the center wavelength of the first fluorescence is B, and the wavelength when the average reflectance of s-polarized light and p-polarized light is 50% due to the reflective film characteristics of a dichroic mirror is cut-off wavelength C, an optical arrangement in which light is incident on a dichroic mirror having the characteristic of A-C < C-B, and a detector that receives the second fluorescence is disposed at the reflection position is considered (FIG. 21).

Light of a desired wavelength at the detector 158 is the first fluorescence, which is mixed with the second fluorescence that can become noise light, and thus the unnecessary second fluorescence needs to be cut off using the bandpass filter 156. On the other hand, light of a desired wavelength at the detector 168 is the second fluorescence, and the first fluorescence that may become noise light is weak, and thus a highly accurate signal can be acquired without the need to provide a bandpass filter. If the weak noise light still poses a problem, a bandpass filter that cuts off the weak first fluorescence may be provided in front of the condenser lens 167, although this is not shown in FIG. 21.

On the other hand, when the detector that receives the second fluorescence is provided at the transmission position of the dichroic mirror 155 and the detector that receives the first fluorescence is provided at the reflection position, the detector provided at the reflection position mainly receives only the second fluorescence, and does not receive the desired first fluorescence. Therefore, the detector that receives the second fluorescence needs to be provided at the reflection position of the dichroic mirror 155.

Next, the case in which a dichroic mirror having film characteristics that satisfy the condition of A-C > C-B is used in the optical system of FIG. 21 is considered. In this case, since the light received by the detector 168 is mixed with the first fluorescence, a bandpass filter (not shown) that cuts off the first fluorescence needs to be provided in front of the detector 168.

As described above, the detector that receives long-wavelength light is provided at the reflection position of the dichroic mirror 155, and if the received light is mixed with short-wavelength light, which is noise light, a bandpass filter that cuts off the short-wavelength light is provided in front of the detector.

(Alternatives to Example 2)

Although a method of measuring the concentration of a measurement object from changes in the emission intensities of two different fluorophores has been described above, a method of measuring the concentration of a measurement object from the polarization anisotropy of two different fluorophores is also possible. Alternatively, a method of measuring the concentration of a measurement object from the polarization anisotropy of one fluorophore and the emission intensity of the other fluorophore is also possible.

The former method of measuring the concentration of a measurement object from the polarization anisotropy of two different fluorophores uses almost the same arrangement as the optical system in FIG. 5. However, to measure the polarization anisotropy of two different fluorophores, a dichroic mirror needs to be provided at the reflection position of the PBS as well as the transmission position of the PBS to separate the fluorescences of different wavelengths. In this case, an additional detector needs to be provided to receive the reflected light of the separated light. However, as described in alternative 5 of example 1, by adding control to alternately or sequentially turn on light sources of different wavelengths, the number of detectors can be reduced and the optical system can be simplified.

The latter method of measuring the concentration of a measurement object from the polarization anisotropy of one fluorophore and changes in the emission intensity of the other fluorophore uses almost the same arrangement as the optical system in FIG. 19, the polarization anisotropy of one fluorophore is calculated from the intensities $I_{para}$ and $I_{orth}$ received by the detectors 48 and 58, and the changes in the emission intensity of the other fluorophore is measured by the detector 68. In this case, the polarizer 64 provided in front of the detector 68 is not necessary, and a polarizer (not shown) after the light source that excites the fluorescence received by the detector 68 is also not necessary.

In this example, an LED or LD is used as a light source, but it is also possible to use an SLD with higher output than an LED, a solid-state laser that emits YAG harmonics, or a gas laser such as He-Ne, which has an excitation wavelength suitable for the emission wavelength of fluorophore.

[Example 3]

(Automated Analytical Apparatus Incorporated Into Rotating Disk)

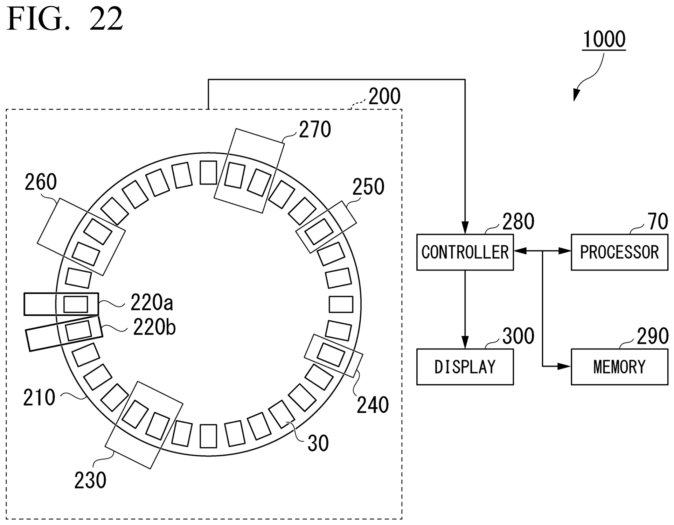

FIG. 22 is a block diagram of an automated analytical apparatus 1000 in this example. As disclosed in Japanese Patent No. 5908954, the automated analytical apparatus 1000 includes an analysis unit 200 and a controller 280 that controls the analysis unit 200. The controller 280 controls a measurement flow in a photometric unit, receives signals output from the photometric unit, and controls a processor 70 and a memory 290 to transfer, process, and store data. The automated analytical apparatus 1000 also includes a display 300 that displays the results processed by the processor 70.

The controller 280 realizes functions thereby by, for example, a hardware processor (computer) executing a program stored in the memory 290. The hardware processor includes, for example, a CPU. The controller 280 is an example of a "controller." That is, the controller 280 simultaneously, alternately, or sequentially turns on at least two light sources that emit light of different wavelengths. A detection unit simultaneously, alternately, or sequentially receives first emitted light and second emitted light depending on the turn-on times of the light sources.

The memory 290 is realized by, for example, a semiconductor memory element such as a random access memory (RAM) or a flash memory, a hard disk, an optical disc, or the like. The memory 290 stores, for example, various types of data related to measurement (such as calibration curves). Such data may be stored in an external memory with which the automated analytical apparatus 1000 can communicate, instead of (or in addition to) the memory 290. The external memory is controlled by a cloud server that manages the external memory and accepts read/write requests, for example.

The display 300 is, for example, a liquid crystal display, a cathode ray tube (CRT), or an organic electroluminescence (EL) display. The display 300 may also be a display device (e.g., a tablet terminal) capable of wireless communication with the automated analytical apparatus 1000.

The analysis unit 200 includes, for example, a rotatable disk 210 and a plurality of reaction vessels 30 disposed on the circumference of the disk 210. The photometric unit may be composed of a photometric unit 220a that performs fluorescence polarization and a photometric unit 220b that performs scattering. The analysis unit 200 also includes a first dispensing unit 230 that dispenses samples such as standard samples and measurement objects into the reaction vessels 30, a first reagent dispensing unit 240 that dispenses a first reagent that reacts with components contained in a sample, and a second reagent dispensing unit 250 that dispenses a second reagent that pairs with the first reagent. The analysis unit 200 also includes a stirring unit 260 that stirs a mixture of a sample and a reagent, and a washing and drying unit 270 that aspirates the mixture from the reaction vessels 30 after measurement, and washes and dries the inside of the reaction vessels 30. Therefore, the automated analytical apparatus 1000 can continuously perform a series of flows of dispensing, stirring, measuring, aspirating, washing, and drying samples and reagents, while rotating the disk 210. The reaction vessels 30 are housed in a thermostatic chamber, maintaining a constant temperature for a reaction solution.

In the above-described apparatus configuration, measurement and analysis are performed according to the flow shown in FIG. 12. In this example, measurement is performed at timing at which the reaction vessel 30 passes through each of the photometric units 220a and 220b. Measurement is performed multiple times while the disk 210 is rotating as a function of reaction time to evaluate anisotropy and the depolarized component of scattering. As in this example, fluorescence polarization and scattering may be performed almost simultaneously and independently. Furthermore, the switching point between fluorescence polarization and scattering, and the calibration curves corresponding to these measurement methods are stored in the memory 290 for post-measurement processing, and the controller 280 reads and executes conditions as needed according to the flow shown in FIG. 12.

The photometric unit may further include a photometric unit 22c (not shown) that performs transmission. Furthermore, as described in example 1, a plurality of combinations are possible, such as using the single photometric unit 220a to perform fluorescence polarization and scattering, and using the single photometric unit 220b to perform transmission.

As described above, the present invention desirably uses a reagent capable of being used for measurements by both fluorescence polarization and scattering, and adjusts the amount of reagent such that the measurement ranges of fluorescence polarization and scattering overlap. As an advantage of the present invention, a wide concentration range can be measured through a single flow shown in FIG. 12 by appropriately selecting and analyzing a plurality of measurement results of measurement methods including scattering and/or transmission while maintaining high sensitivity using fluorescence polarization. Scattering is a means for measuring scattered light, and as described above, several methods are conceivable, such as a depolarized component of non-excitation light, the intensity of light emitted at a certain scattering angle, or an autocorrelation function calculated from temporal fluctuations in the scattered light intensity. Further, transmitted light intensity measured using transmission may also be included.

The object of the present invention is to expand the range of concentrations that can be measured by combining a measurement method that uses fluorescence polarization to measure agglutination due to an antigen-antibody reaction as changes in rotational motion with a measurement method that uses scattering to measure changes in scattering cross section or translational motion. The former is volume-dependent and therefore, in principle, more sensitive to minute changes than the latter. Utilizing this principle, agglutination changes due to an antigen-antibody reaction are measured independently using methods based on two different physical phenomena. The measurement range can be expanded by appropriately selecting two measurement results depending on the concentration of a measurement object.

Although the embodiments of the present invention have been described with reference to exemplary embodiments, it should be understood that the present invention is not limited to the above-described embodiments. The scope of the appended claims should be construed in the broadest sense to encompass all such modifications and equivalent structures and functions.

## Claims

1. An automated analytical apparatus comprising:

   a light source unit that emits at least two lights having different wavelengths;
   a reaction vessel that contains a reaction solution containing a mixture of a measurement object and a reagent specifically reacting with the measurement object;
   a detection unit that receives at least two emitted lights of first emitted light and second emitted light having different wavelengths emitted from the reaction vessel by radiating incident light emitted from the light source unit to the reaction vessel; and
   a processor configured to calculate a concentration of the measurement object based on a signal output from the detection unit,
   wherein the first emitted light is fluorescence obtained by wavelength conversion of the incident light by the reagent,
   the detection unit has a separation means for separating the first emitted light and the second emitted light and receiving the separated first emitted light and second emitted light through photodetectors, and
   the processor is configured to calculate the concentration of the measurement object from at least one of two output signals output from the detection unit in response to the first emitted light and the second emitted light.

2. The automated analytical apparatus according to claim 1, wherein the processor is configured to:

   evaluate signal changes in the output signals after a given time has elapsed since the start of a reaction in which the measurement object and the reagent are mixed;

calculate a first signal change based on the first emitted light and a second signal change based on the second emitted light; and

calculate the concentration of the measurement object based on the first signal change when the first signal change satisfies a predetermined condition, and calculate the concentration of the measurement object based on the second signal change when the first signal change does not satisfy the predetermined condition.

3. The automated analytical apparatus according to claim 2, wherein

the reagent includes scattering particles containing fluorescent molecules,

the first signal change is a signal change based on fluorescence anisotropy, and

the second signal change is a signal change based on scattered light intensity or an autocorrelation function calculated from temporal fluctuations in scattered light intensity.

4. The automated analytical apparatus according to claim 2, wherein

the reagent contains at least two antibodies with different affinities, each labeled with a fluorescent molecule that emits light at a different wavelength, and

the first signal change and the second signal change are signal changes based on a fluorescence intensity of the fluorescence having a different wavelength.

5. The automated analytical apparatus according to any one of claims 1 to 4, wherein the separation means of the detection unit is a dichroic mirror that reflects light having a longer wavelength of two lights having different wavelengths and transmits light having a shorter wavelength.

6. The automated analytical apparatus of any one of claims 1 to 3, wherein

the light source unit has a linear polarizer, and at least one of the lights emitted from the light source unit is linearly polarized and irradiates the reaction solution, and

the detection unit has a polarization separation element, separates the first emitted light emitted from the reaction solution by the linearly polarized light into a direction parallel to and a direction orthogonal to a polarization direction of the linearly polarized light, and receives the separated lights through the photodetectors.

7. The automated analytical apparatus according to claim 5, wherein desired light received by the photodetector is incident on the dichroic mirror as s-polarized light and color-separated when A-C < C-B and incident on the dichroic mirror as p-polarized light and color-separated when A-C > C-B, C being a cutoff wavelength at which the reflectance of the dichroic mirror is 50%, A being the wavelength of light having the longer wavelength of the two lights having different wavelengths, and B being the wavelength of the light having the shorter wavelength.

8. The automated analytical apparatus according to any one of claims 1 to 4, wherein, with respect to light intensities of the photodetector receiving the first emitted light and the photodetector receiving the second emitted light, an output of a first light source generating an emitted light with a lower light intensity is greater than an output of a second light source, or a gain or exposure time of the photodetector receiving the emitted light with a lower light intensity is greater than that of the other photodetector.

9. The automated analytical apparatus according to any one of claims 1 to 4, further comprising a controller configured to alternately or sequentially turn on at least two light sources emitting lights having different wavelengths,

wherein the detection unit alternately or sequentially receives the first emitted light and the second emitted light depending on turn-on times of the light sources.

10. The automated analytical apparatus according to any one of claims 1 to 4, wherein the lights emitted from the light source unit are incident through a first surface of the reaction vessel and emitted through a second surface opposite the first surface.

11. The automated analytical apparatus according to claim 2 or 3, wherein the predetermined condition includes comparing the first signal change with the second signal change, and calculating the concentration of the measurement object based on concentration change of the measurement object or the larger signal change with respect to the elapse of time.

12. An automated analytical method comprising:

a radiation step of radiating at least two lights having different wavelengths to a reaction solution containing a mixture of a measurement object and a reagent specifically reacting with the measurement object;

a light-receiving step of separately receiving at least two emitted light of a first emitted light and a second emitted light having different wavelengths emitted from the reaction solution; and a processing step of calculating a concentration of the measurement object based on a signal output in the light-receiving step, wherein the processing step calculates the concentration of the measurement object from at least one of a change in the signal based on the first emitted light and a change in the signal based on the second emitted light.

FIG. 1

FIG. 2

FIG. 3

FIG. 4

FIG. 5

EP 4 737 887 A2

# FIG. 6

SPECTRUM OF LIGHT EMITTED FROM REACTION VESSEL 30

# FIG. 7

FILM CHARACTERISTICS OF DICHROIC MIRROR 55

# FIG. 8

SPECTRUM AFTER SEPARATION BY DICHROIC MIRROR 55 IN FIG. 5

# FIG. 9

OTHER FILM CHARACTERISTICS OF DICHROIC MIRROR 55

# FIG. 10

SPECTRUM AFTER SEPARATION BY DICHROIC MIRROR 55 IN FIG. 5
(WHEN DICHROIC MIRROR HAVING FILM CHARACTERISTICS SHOWN IN FIG. 9 IS USED)

# FIG. 11

# FIG. 12

START

S1
DISPENSE MEASUREMENT OBJECT AND REAGENT

S2
MEASURE ANISOTROPY r OF FLUORESCENCE AND DEPOLARIZED COMPONENT OF SCATTERED LIGHT

S3
HAS REACTION TIME ELAPSED?
NO
YES

S4
ANISOTROPY $r \geq r_1$?
NO
YES

S5
CALCULATE CONCENTRATION OF MEASUREMENT OBJECT

S6
ANISOTROPY $r \geq r_{MAX}$?
NO
YES

S8
CALCULATE CONCENTRATION OF MEASUREMENT OBJECT

S7
CALCULATE CONCENTRATION OF MEASUREMENT OBJECT

END

FIG. 13

(a)
FLUORESCENCE POLARIZATION
(MEASUREMENT OBJECT CONCENTRATION $\rho 1$)

ANISOTROPY

$r_1$

REACTION TIME

(b)
FLUORESCENCE POLARIZATION
(MEASUREMENT OBJECT CONCENTRATION $\rho 2 (> \rho 1)$)

ANISOTROPY

$r_1$

REACTION TIME

(c)
SCATTERING
(MEASUREMENT OBJECT CONCENTRATION $\rho 1$)

SIGNAL

REACTION TIME

(d)
SCATTERING
(MEASUREMENT OBJECT CONCENTRATION $\rho 2 (> \rho 1)$)

SIGNAL

REACTION TIME

# FIG. 14

# FIG. 15

SPECTRUM AFTER SEPARATION BY DICHROIC MIRROR 55 IN FIG. 14

# FIG. 16

SPECTRUM AFTER SEPARATION BY DICHROIC MIRROR 55 IN FIG. 14
(WHEN DICHROIC MIRROR HAVING FILM CHARACTERISTICS SHOWN IN FIG. 9 IS USED)

# FIG. 17

FIG. 18

FIG. 19

FIG. 20

FIG. 21

EP 4 737 887 A2

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1692254 A
- JP 6013796 B
- JP 2007120976 A
- JP 5908954 B